# EUROPEAN PATENT SPECIFICATION

(11) **EP 2 046 405 B1**
(45) Date of publication and mention of the grant of the patent: **05.09.2012**
(21) Application number: 07787800.7
(22) Date of filing: 23.07.2007
(51) Int. Cl.: A61Q 11/00, C04B 35/22, A61K 6/027, C03C 3/04, C04B 35/057, C04B 35/14, C04B 35/447, C04B 38/00, A61K 8/25, A61L 27/10, A61L 27/42, A61L 27/56, C03C 1/00, C03C 3/00, C03C 4/00

(54) **BIOMATERIALS, THEIR PREPARATION AND USE**
BIOLOGISCHE MATERIALIEN, IHRE HERSTELLUNG UND VERWENDUNG
BIOMATÉRIAUX, LEUR PRÉPARATION ET LEUR UTILISATION

(30) Priority: 01.08.2006 WO PCT/CN2006/001932
(43) Date of publication of application: 15.04.2009
(73) Proprietor: Unilever PLC, London EC4Y 0DY (GB); Unilever N.V., 3013 AL Rotterdam (NL)
(72) Inventor: BUTLER, Michael Francis, Bedford Bedfordshire MK44 1LQ (GB); HEPPENSTALL-BUTLER, Mary, Bedford Bedfordshire MK44 1LQ (GB); DENG, Yan, Shanghai 200051 (CN); SHEN, Shaodian, Colombus, Ohio 43210 (US); ZHU, Guibo, Shanghai 200051 (CN)
(74) Representative: James, Helen Sarah
(86) International application number: PCT/EP2007/057556
(87) International publication number: WO 2008/015117

(56) References cited:
- WO-A-02/096391
- US-B1- 6 482 444
- DATABASE WPI Week 200668 Derwent Publications Ltd., London, GB; AN 2006-649733 XP002477844 & CN 1 785 862 A (SHANGHAI INST SILICATE CAS) 14 June 2006 (2006-06-14)
- DATABASE WPI Week 200521 Derwent Publications Ltd., London, GB; AN 2005-197251 XP002477845 & CN 1 554 607 A (UNIV FUDAN) 15 December 2004 (2004-12-15)
- HORCAJADA P ET AL: "Bioactivity in ordered mesoporous materials" SOLID STATE SCIENCES, ELSEVIER, PARIS, FR, vol. 6, no. 11, November 2004 (2004-11), pages 1295-1300, XP004612079 ISSN: 1293-2558
- DIAZ ET AL: "Growth of hydroxyapatite in a biocompatible mesoporous ordered silica" ACTA BIOMATERIALIA, ELSEVIER, AMSTERDAM, NL, vol. 2, no. 2, March 2006 (2006-03), pages 173-179, XP005290696 ISSN: 1742-7061
- ZHAO D ET AL: "Triblock Copolymer Syntheses of Mesoporous Silica with Periodic 50 to 300 Angstrom Pores" SCIENCE, AMERICAN ASSOCIATION FOR THE ADVANCEMENT OF SCIENCE,, US, vol. 279, 23 January 1998 (1998-01-23), pages 548-552, XP002271540 ISSN: 0036-8075
- YAN, YU, ZHOU, TANG, ZHAO: "highly ordered mesoporous bioactive glasses with superior in vitro bone-forming bioactivities" ANGEW. CHEM. INT. ED, vol. 43, 2004, pages 5980-5984, XP002476843 cited in the application

## Description

### FIELD OF THE INVENTION

The present invention relates to calcium oxide-silica composite biomaterials having a particular average pore size, to methods for their preparation and to uses thereof.

### BACKGROUND

Apatite is a mineral that is produced and used by biological systems. The name apatite refers to a group of phosphate minerals, including hydroxylapatite, fluoroapatite and chloroapatite (having high concentrations of hydroxyl, fluoride and chloride anions respectively in the crystal lattice). The formula of the admixture of the three most common species of apatite is Ca₅(PO₄)₃(OH, F, Cl).

Hydroxylapatite is the major component of tooth enamel and a large component of bone material. It is a naturally occurring form of apatite, having the formula Ca₅(PO₄)₃OH (usually written as Ca₁₀(PO₄)₆(OH)₂, to denote that the crystal unit cell comprises two molecules).

Typically, hydroxylapatite has a prism-like shape with a width of about 60 nm and a length of several micrometers. The prism crystallites generally are aligned in a highly ordered manner.

It is known to use hydroxylapatite as a bone replacement material and as a coating on metal implants to promote bone in-growth, for example into prosthetic implants. Newly formed hydroxylapatite on implant surfaces helps to stimulate cells to secrete growth factors and to promote new tissue growth to form good bonding with implants. It is also known to use hydroxylapatite where remineralisation is required, for example in remineralisation of tooth enamel and bone, i.e. to treat osteoporosis. By the term "remineralisation", we mean restoring of depleted mineral content.

There has, therefore, been considerable interest in providing compositions for and methods of inducing hydroxylapatite formation.

Crystallised hydroxylapatite may be formed by sintering various calcium phosphate compounds at a given ratio and at a temperature above 600°C. The crystallised hydroxylapatite may then be ground into powder and blended with a polymer matrix, for use as a dental or bone implant. In use, the crystallised hydroxylapatite is dissolved in body fluid and induces the formation of new hydroxylapatite on or in a tooth or bone. However, this process is very slow and the formation of new hydroxylapatite takes a long time, often from several months to a year.

In an attempt to speed up the formation of hydroxylapatite a new type of cement system was developed. For example, US-4,612,053 describes a cement system that comprises tetracalcium phosphate (Ca₄(PO₄)₂O) and at least one other sparingly soluble calcium phosphate solid, such as dicalcium phosphate anhydrous (CaHPO4₎. The cement forms hydroxylapatite when it is mixed with sodium phosphate solution. However, the cement produces a lot of heat upon hydroxylapatite formation, which harms surrounding tissue. Additionally, the cement is costly and difficult to make.

Porous biomaterials have also been developed in an attempt to enhance bioactivity and new bone in-growth.

Porous materials are classified into several kinds according to their size. For example, microporous material have pore diameters of less than 2 nm, mesoporous materials have pore diameters between 2 and 50 nm and macroporous materials have pore diameters of greater than 50 nm.

An example of a type of porous biomaterial is a porous bioactive glass.

Bioactive glasses comprise SiO₂, CaO, P₂O₅, Na₂O and small amounts of other oxides. A bioactive glass typically has the basic formula CaO-P₂O₅-Na₂O-SiO₂. Bioactive glasses may be made by melt processes or by sol-gel processing (see, for example, Hench, J. Am, Ceram. Soc., 81, 7, 1705-28, (1998) and Hench, Biomaterials, 19 (1998), 1419-1423).

Horcajada et al (Solid State Sciences 6 (2004) 1295-1300), describes how the addition of glass to MCM-41 (a mesoporous silica having a hexagonal porous structure) induces the formation of an a pactite-like layer starting from the glass surface.

Bioactive glasses are known to bond to living bone. When a bioactive glass is immersed in body fluid, it is believed that calcium and phosphate ions migrate from the bioactive glass so as to form a calcium-phosphate rich surface layer. The layer below the calcium-phosphate rich surface becomes increasingly silica rich due to the loss of calcium ions. Upon exposure to water, silica forms Si-OH bonds. The hydroxyl group attracts calcium ions and the calcium ions attract phosphate ions so as to precipitate and transform into more stable hydroxylapatite (as suggested by Kokubo, "Apatite formation on surface of ceramics metals", Acta mater., Vol. 46, No. 7, 2519-2527, 1998). Thus, a layer of hydroxylapatite is formed on the bioactive glass. Cells then adhere to the layer of hydroxylapatite and gradually attach firmly to the bioactive glass so as to lay down an excelluar matrix. The matrix mineralises so as to connect with the bone tissue. Thus the bone bonds to the bioactive glass.

US-B-6,338,751 describes a bioactive glass composition including particulate bioactive and biocompatible glass containing 40 to 60% SiO₂, 10 to 30% CaO, 10 to 35% Na₂O, 2 to 8% P₂O₅, 0 to 25% CaF₂ and 0 to 10% B₂O₃ (where are percentages are by weight) and a particle size range less than 90 µm and including an effective dentin tubule occluding amount of particles less than about 10 µm.

US-A-2004/0087429 describes a bioactive glass comprising 30 to 60 mol% CaO, 40 to 70 mol% SiO₂ and 20 mol% or less Na₂O and its use in bone restoration materials.

WO-A-2005/063185 describes non-aqueous compositions comprising bioactive glass particles. The bioactive glass may comprise from 40 to 86% SiO₂, from 0 to 35% Na₂O, from 4 to 46% CaO and from 1 to 15% P₂O₅ (where are percentages are by weight).

CN-1554607 describes mesoporous and macroporous biological glass produced through surfactant self-assembling and sol-gel processes using surfactants and polymer beads. The surfactants used in the processes are E0₂₀PO₇₀EO₂₀ (P123), EO₁₀₆PO₇₀EO₁₀₆ (F127), EO₁₃₂PO₅₀EO₁₃₂ (F108), EO₂₀PO₃₀EO₂₀ (P65) and EO₂₆PO₃₉EO₂₆ (P85), wherein EO is poly(ethylene)oxide and PO is poly(propylene)oxide. The polymer beads used in the processes are polystyrene and polybutyl methacrylate. The glasses produced include phosphate ions. The glasses produced using the surfactants P123, P65 and P85 have average pore sizes of 4.6 nm, 5.1 nm and 6.0 nm respectively.

CN-1785862 describes a calcium oxide-silica composite biomaterial obtained by a sol gel process in the presence of a surfactant as directing agent. The average pore size of the composites is between 5 and 10nm, the calcium oxide silica content is at least 80 wt% and the molar ratio of calcium oxide to silica is at least 0.1. The composite do not contain phosphate ions in an amount superior to 0.5wt%. Furthermore, the porosity has a ordered arrangement.

Hench et al. (Journal of Sol-Gel Science and Technology, 7, 59-68, 1996) describes gel-silica glasses having different pore sizes and teaches that the larger pore sizes are preferred.

Yu et al. (Angew. Chem. Int. Ed., 2004, 43, 5980-5984) describes a process for making highly ordered mesoporous bioactive glasses. The process comprises dissolving a nonionic block copolymer, tetraethyl orthosilicate (TEOS), calcium nitrate, triethyl phosphate and hydrochloric acid in ethanol and stirring the solution at room temperature to produce a sol. The sol then undergoes an evaporation-induced self-assembly (EISA) process and the dried gel is calcined at 700°C to obtain the mesporous bioactive glass. The nonionic block polymers are used as structure-directing agents to provide the desired pore size and structure. The nonionic block copolymers used are EO₂₀PO₇₀EO₂₀ (P123), EO₁₀₆PO₇₀EO₁₀₆ (F127) and EO₃₉BO₄₇EO₃₉ (B50-6600), wherein EO is poly(ethylene)oxide, PO is poly(propylene)oxide and BO is poly(butylene)oxide. The mesoporous glasses formed by this process apparently are homogeneous and have a pore size in the range of from 4 to 7 nm. Yu et al. teaches that the mesoporous glasses formed by this process have superior bone-forming bioactivity *in vitro.*

It is known to use other surfactants as structure-directing agents for forming mesoporous and microporous materials.

For example, cetyltrimethylammonium bromide (CTAB) is known as a porogen molecule (or structure-directing agent) in mesoporous silica (see S. Mann et al., Adv. Mater. 2002, 14, No. 11, June 5, pages 1 to 14).

Stucky et al. (Science, Vol. 279, 1998, pages 548-552) describes the use of cationic cetyltrimethylammonium surfactants to make MCM-41 (a mesoporous silica having a hexagonal porous structure) having uniform pore sizes of from 2 to 3 nm. Stucky et al. also teaches that well-ordered hexagonal mesoporous silica structures with tunable large uniform pore sizes of up to 30 nm can be formed using amphiphilic block copolymers as organic structure-directing agents.

Holmberg et al. (Soft Matter., 2005, 1, 219-2-26) describes the use of cationic and nonionic surfactants as structure-directing agents to make mesoporous silica.

None of the aforementioned prior art documents disclose a calcium oxide-silica composite biomaterial as defined below or a method for preparing such calcium oxide-sitica composite biomaterials.

### SUMMARY OF THE INVENTION

A first aspect of the present invention provides a calcium oxide-silica composite biomaterial either in amorphous state or crystalline state having an average pore size, as determined by the BET method, in the range of from 0.8 to 4 nm, wherein the calcium oxide-silica content of the biomaterial is at least 80 wt %, the balance being optionally one or more other materials and wherein the molar ratio of calcium oxide to silica is at least 0.1 and wherein the biomaterial includes less than 0.5% by weight of phosphate ions and wherein the pores of the biomaterial have an ordered arrangement.

A second aspect of the present invention provides a method for preparing a calcium oxide-silica composite biomaterial as defined above, the method comprising the steps of:
(i) combining, in solution, a calcium salt, an organic or inorganic silica precursor such as a silicate or a tetra(alkyl)silicate and a structure-directing agent in the presence of an aqueous solvent whereby hydrolysis of the tetra(alkyl)silicate occurs, leading to the formation of a sol;
(ii) isolating a solid from the sol; and
(iii) calcinating the isolated solid.

The calcium oxide-silica composite biomaterials of the present invention provide very real advantages in use. For example, they are easy to prepare and are capable of inducing the formation of hydroxylapatite over a much shorter period of time than the prior art biomaterials discussed above. Uses include tissue regeneration, tooth and/or bone regeneration, tooth whitening and treating and/or preventing tooth hypersensitivity.

### DETAILED DESCRIPTION OF THE INVENTION

Unless defined otherwise, all technical and scientific terms used herein have the same meaning as commonly understood by one of ordinary skill in the art.

### Calcium Oxide-Silica Composite Biomaterials

According to the present invention, there is provided a calcium oxide (CaO)-silica (SiO₂) composite biomaterial as defined above.

For the avoidance of any doubt, by the term "composite" we mean a single material formed of at least two different materials. The calcium oxide-silica composite biomaterials of the present invention comprise at least calcium oxide and silica. In other words, the calcium oxide-silica composite biomaterials comprise at least calcium, oxygen and silicon atoms bonded together to form the biomaterials.

As the skilled person would appreciate, the calcium oxide-silica composite biomaterials of the present invention may comprise additional components (i.e. in addition to calcium oxide and silica). Any such additional components may be included, provided that the biomaterial includes less than 0.5% by weight of phosphate ions, and provided that they do not inhibit or prevent the calcium oxide-silica composite biomaterials of the present invention from inducing the formation of hydroxylapatite as discussed in more detail below. In other words, it is preferred that any additional components do not participate in and/or inhibit the action of the calcium oxide-silica composite biomaterials in forming hydroxylapatite. This is because the present inventors believe that calcium oxide and silica alone are particularly effective in inducing the formation of hydroxylapatite in a solution containing phosphate ions, for example a body fluid or saliva. Any additional components may, for example, be included in an amount of less than 15% by weight, more preferably of less than 10% by weight.

For the avoidance of any doubt, by the term "biomaterial" we mean a material that is capable of bonding to human and/or animal tissue, including living tissue (such as bone tissue and tooth dentin) and non-living tissue (such as tooth enamel) and also including both soft and hard tissue.

The average pore size is that measured using the BET method. This may be performed using a commercially available instrument.

In another aspect of the present invention, the average pore size is in the range of from 2 to 4 nm, particularly in the range of from 2 to less than 4 nm, for example in the range of from 2 to 3.9 nm, particularly in the range of from 2 to 3.5 nm, more particularly in the range of from 2 to 3 nm.

In another aspect of the present invention, the average pore size is in the range of from 1 to 2.7 nm and in yet another aspect of the present invention, the average pore size is in the range of from 1.35 to 2.45 nm.

As the skilled person would appreciate, it is not essential for 100% of the pores to be of the specified pore size in order for the calcium oxide-silica composite biomaterials of the present invention to exhibit the advantages discussed above. The term "average pore size" is widely used in the art and would be understood by a person skilled in the art. The average pore size is calculated by a known statistical method.

The average pore size is controlled and selected by the use of an appropriate structure-directing agent during the formation of the calcium oxide-silica composite biomaterial, for example using the method described herein. In other words, the structure-directing agent is selected so as to provide the desired average pore size. As the skilled person would appreciate, the particular average pore size obtained depends on the particular structure-directing agent used. Other factors may also affect the average pore size, such as, for example, the pH and temperature of the preparation solution and the concentration of the structure-directing agent.

The calcium oxide-silica composite biomaterials of the present invention are in the amorphous state or crystalline state. Mixtures of biomaterials of both states are also within the ambit of the invention. Amorphous state includes the glass state.

The calcium oxide-silica composite biomaterials of the present invention typically are silica-based materials. In other words, the biomaterials comprise a primary structure of silica, i.e. interconnected silicon and oxygen atoms. The particular structure formed by the network of interconnected silicon and oxygen atoms may be any suitable structure and will depend on several factors, including the nature of the structure-directing agent used to prepare the composite biomaterial. For example, when the structure-directing agent is CTAB typically a hexagonal porous structure is formed and when the structure-directing agent is F127® typically a cubic porous structure is formed. Calcium atoms are covalently bonded to the oxygen atoms in the silicon-oxygen network, so as to form a coherent and continuous mix of silicon, oxygen and calcium atoms. The composite material typically has a spherical shape once formed.

The pores of the calcium oxide-silica composite biomaterial have an ordered arrangement. The ordering of the pores can, for example, be detected by small angle X-ray diffraction, for example at angles of from 1 to 8° (compared to angles of 10 to 80° used for a normal crystal). Small angle X-ray diffraction is required because the pore size is larger than the atom crystal lattice. As the skilled person would appreciate, if the pores do not have an ordered arrangement, no peaks are observed in the small angle X-ray diffraction pattern. If, however, the pores have an ordered arrangement, a sharp peak is observed in the X-ray diffraction pattern.

The present inventors surprisingly have found that the calcium oxide-silica composite biomaterials of the present invention are especially effective at inducing the formation of hydroxylapatite, for example compared to the prior art biomaterials discussed above.

As discussed above, it is believed that in order for hydroxylapatite to form, calcium ions must be released from an appropriate biomaterial. In particular, in order for hydroxylapatite to form on the surface of a biomaterial, calcium ions must migrate to the surface of the material. Without wishing to be bound by any theory, it is believed that the average pore sizes of the calcium oxide-silica composite biomaterials of the present invention, which are small compared to many known biomaterials, provide a higher inner surface area, which allows for easy and efficient dissolution of the calcium atoms. Typically, the inner surface area of the calcium oxide-silica composite biomaterials of the present invention is in the range of from 400 to 1000 m²/g. Furthermore, the calcium oxide-silica, composite biomaterials of the present invention include a well-ordered arrangement of pores and channels. Without wishing to be bound by any theory, it is believed that this well-ordered arrangement allows easy transport of the calcium ions to the surface of the biomaterial, so as to aid the formation of hydroxylapatite. Additionally, it is believed that the small pore sizes prevent or reduce the formation of hydroxylapatite in the pores, so as to avoid blockage of the pore channel and thus increase the amount of hydroxylapatite that is formed at the surface, as desired.

The calcium oxide-silica composite biomaterials of the present invention may comprise calcium and silicon in any suitable ratio, provided that the molar ratio of calcium oxide to silica is at least 0.1. For example, the molar ratio of calcium to silicon may be in the range of from 1:10 to 1:1, for example in the range of from 1:10 to 1:2, particularly about 1:10. It is believed that this molar ratio helps to control the rate of release of calcium atoms from the composite biomaterial and, as the skilled person would appreciate, the optimum molar ratio will depend on the particular composite biomaterial and the conditions under which it is used.

The calcium oxide-silica composite biomaterials of the present invention include less than 0.5% by weight, of phosphate ions,. For example, it is possible to prepare a calcium oxide-silica biomaterial of the present invention containing less than 0.005% by weight of phosphate ions using high purity starting materials, for example using calcium nitride supplied by China National Pharmaceutical Group Corporation (SINOPHARM), Beijing, China in a purity of greater than 99%.

The calcium oxide-silica composite biomaterials of the present invention that are substantially free of phosphate ions are believed to be advantageous because, in use, the formation (and precipitation) of calcium phosphate in the pores of the biomaterial is reduced. Instead, the calcium ions are able to migrate to the surface of the biomaterial before combining with the phosphate ions from aqueous solution to form calcium phosphate. This aids the formation of hydroxylapatite at the outer surface of the biomaterial. These biomaterials are in contrast to conventional biomaterials that included phosphate ions in their structure. Additionally, the calcium oxide-silica composite biomaterials of the present invention that are substantially free of phosphate ions are believed to be advantageous because they have a simple composition and are easy to prepare.

The composite biomaterials of the present invention may contain one or more other materials provided, that the calcium oxide-silica content is at least 80 wt%. The other material(s) are typically selected from those commonly found in bioglasses, provided that the biomaterial includes less than 0.5% by weight of phosphate ions, as described above.

As discussed above, in many applications it is preferred to form the hydroxylapatite at the surface of the biomaterial. This allows for the subsequent cascade of physiochemical interactions to occur that are required to form a bond to the tissue. Examples of such application include coatings on metal implants, for example on titanium oxide implants.

Typically, the calcium oxide-silica composite biomaterials of the present invention are in the form of a powder. This is advantageous because it allows the materials to be used in powder form without requiring the step of forming a powder, for example by grinding into a powder form. Furthermore, the powder typically comprises particles of a small size that cannot readily be obtained by simple grinding processes, for example of a submicron size.

In one aspect of the invention, the calcium oxide-silica composite biomaterials of the present invention may be in the form of a glass. In order to form a glass, a suitable calcination temperature should be used, for example a calcination temperature of at least 900°C.

### Method

According to the present invention, there is provided a method for preparing a calcium oxide-silica composite biomaterial as defined above, the method comprising the steps of:
(i) combining, in solution, a calcium salt, a tetra(alkyl)silicate and a structure-directing agent in the presence of an aqueous solvent whereby hydrolysis of the tetra(alkyl)silicate occurs, leading to the formation of a sol:
(ii) isolating a solid from the sol; and
(iii) calcinating the isolated solid.
With above described method it is also possible to produce calcium oxide-silica composite biomaterial having an average pore size in the range of from 2 to 4 nm, particularly in the range of from 2 to less than 4 nm, for example in the range of from 2 to 3.9 nm, particularly in the range of from 2 to 3.5 nm, more particularly in the range of from 2 to 3 nm.

With above described method it is also possible to produce calcium oxide-silica composite biomaterial having an average pore size of from 1 to 2.7 nm and in yet another aspect of the present invention, the average pore size is in the range of from 1.35 to 2.45 nm.

In step (i) of the method of the present invention, the tetra(alkyl)silicate (such as TEOS) is hydrolysed to form silica. As the skilled person would appreciate, it is not necessary for all of the tetra(alkyl)silicate to be hydrolysed. Typically at least 80% by weight of the tetra(alkyl)silicate is hydrolysed in step (i).

As the skilled person would appreciate, any suitable tetra(alkyl)silicate may be used in step (i) of the method of the present invention. Suitable tetra(alkyl)silicates include tetraethyl orthosilicate (hereinafter referred to as "TEOS") and tetramethyl orthosilicate. It is less preferred to use tetramethyl orthosilicate because tetramethyl orthosilicate produces methanol during the hydrolysis reaction. Methanol is known to be harmful to humans and animals. Also, methanol potentially may disrupt the formation of the ordered structure in the sol.

Any suitable concentration of tetra(alkyl)silicate may be used in step (i) of the method of the present invention. Suitable concentrations include 0.1 to 1M, particularly 0.3 to 0.6M.

As the skilled person would appreciate, any suitable calcium salt may be used in step (i) of the method of the present invention. Suitable calcium salts include those that are substantially soluble in an aqueous solution with a pH between 8 and 10. For example, suitable calcium salts include calcium nitrate, and calcium chloride. In one aspect, the calcium salt is calcium nitrate.

It is possible in step (i) of the method of the present invention, for some of the calcium salt to hydrolyse to form calcium hydroxide. This typically will only occur at pH values of greater than 8.

Any suitable concentration of calcium salt may be used in step (i) of the method of the present invention. The concentration is selected so as to provide the desired ratio of calcium and silicon, as discussed above.

As the skilled person would appreciate, any suitable structure-directing agent may be used in step (i) of the method of the present invention, provided that it is capable of forming a calcium oxide-silica composite biomaterial having an average pore size in the range specified. For example, the structure-directing agent may be a cationic or a nonionic surfactant and should be organic in nature. Suitable structure-directing agents are disclosed in Berggren et al., Soft Matter., 2005, 1, 219-226.

Suitable structure-directing agents include, for example, cationic surfactants of the general formula CₙH₂ₙ₊₁N(CH₃)₃X, wherein X represents bromo or chloro and n is 8, 10, 12, 14 or 16. When the structure-directing agent is such a surfactant, the calcium oxide-silica composite biomaterial produced typically has an average pore size in the range of from about 1.7 to 2.7 nm.

An example of such a cationic surfactant is cetyltrimethylammonium bromide (hereinafter referred to as "CTAB"), which has the formula C₁₆H₃₃N(CH₃)₃Br (i.e. n is 16). CTAB is commercially available (for example from Acros Organics, New Jersey, USA). When the structure-directing agent is CTAB, the calcium oxide-silica composite biomaterial produced has an average pore size of about 2.7 nm.

Further suitable structure-directing agents include, for example, nonionic surfactants of the general formula CₘH₂ₘ₊₁NH₂, wherein m is 8, 10, 12, 14, 16 or 18. When the structure-directing agent is such a surfactant, the calcium oxide-silica composite biomaterial produced typically has an average pore size in the range of from about 1.6 to 2.4 nm.

An example of such a nonionic surfactant is dodecylamine, which has the formula H₂N(C₁₂H₂₅) (i.e. m is 12). Dodecylamine is commercially available (for example from Tokyo Kasei Kogyo Company Limited, Japan). When the structure-directing agent is dodecylamine, the calcium oxide-silica composite biomaterial produced has an average pore size of about 2.4 nm.

A further suitable structure-directing agent is Pluronic F88®, which is a nonionic block copolymer surfactant of the formula EO₁₀₀PO₃₉EO₁₀₀, wherein EO represents poly(ethylene)oxide and PO represents poly(propylene)oxide. Pluronic F88® is commercially available (for example from BASF Corporation). When the structure-directing agent is Pluronic F88®, the calcium oxide-silica composite biomaterial produced has an average pore size of about 3.5 nm.

A further suitable structure-directing agent is Tetronic 908®, which is a nonionic star copolymer surfactant of the formula (EO₁₁₃PO₂₂)₂N(CH₂)₂N(PO₂₂EO₁₁₃)₂, wherein EO represents poly(ethylene)oxide and PO represents poly(propylene)oxide. Tetronic 908® has an average molecular weight of 25000. Tetronic 908® is commercially available (for example from BASF Corporation). When the structure-directing agent is Tetronic 908®, the calcium oxide-silica composite biomaterial produced has an average pore size of about 3.0 nm.

In one aspect of the present invention, the structure-directing agent is selected from cetyltrimethylammonium bromide (CTAB), Pluronic F88®, Tetronic 908® and dodecylamine, and mixtures thereof. In particular, the structure-directing agent may be CTAB.

Any suitable concentration of structure-directing agent may be used in step (i) of the method of the present invention and will depend on the particular structure-directing agent(s) being used. Suitable concentrations include, for example, 50 to 100 mM when the structure-directing agent is CTAB and 4 to 12 mM when the structure-directing agent is dodecylamine.

As the skilled person would appreciate, mixtures of one or more calcium salts, tetra(alkyl)silicates and/or structure-directing agents may be used in step (i) of the method of the present invention. It is, however, preferred to use only one structure-directing agent, as this aids the formation of pores of the desired size.

The aqueous solvent may comprise water and an alcohol. Any suitable alcohol may be used, for example a C₁-C₄ alcohol such as methanol or ethanol (especially ethanol). The use of ethanol is advantageous because it is inexpensive and is not harmful to health during production.

The water in the aqueous solvent is required in order for the aforementioned hydrolysis reaction(s) to occur. Thus, the aqueous solvent must contain a sufficient amount of water to allow the hydrolysis reaction(s) to occur. Typically the aqueous solvent comprises between 40 and 50% by weight, for example about 45% by weight, of water. The amount of alcohol controls the rate of the hydrolysis reaction(s). As the amount of alcohol is increased, the rate of hydrolysis of the tetra(alkyl)silicate is decreased.

As the hydrolysis reaction(s) proceeds in step (i) of the method of the present invention, the silica and the calcium salt typically form a sol. The hydrolysed tetra(alkyl)silicate typically undergoes a condensation reaction, which leads to the formation of the desired network of interconnected silicon and oxygen atoms. For the avoidance of any doubt, by the term "sol", we mean a dispersion of colloidal particles in a liquid.

Without wishing to be bound by any theory, it is believed that above the critical micelle concentration, the structure-directing agent forms micelles, i.e. spherical or cylindrical structures that maintain the hydrophilic parts of the structure-directing agent in contact with water while shielding the hydrophobic parts within the micellar interior. The micelles then undergo a self-assembly process to form a three-dimensional structure within the sol. By the term "self-assembly" we mean the spontaneous organisation of the micelles through non-covalent interactions, such as hydrogen bonding, Van der Waals forces, electrostatic forces, π-π interactions (see, for example, Advanced Materials, Vol. 11, Issue 7, pages 579 to 585).

The particular three-dimensional structure formed by the micelles depends on several factors, including the interfacial tension energy of the micelles and the remainder of the solution. The interfacial tension energy is directly related to the interfacial area. The smallest interfacial energy is obtained by providing the smallest interfacial area, i.e. by closely packed micelles. The micelles closely pack when they are packed in an ordered manner. Thus, the most ordered packing of the micelles provides the lowest energy state or the thermodynamically favoured state.

Without wishing to be bound by any theory, it is believed that the calcium and silica molecules are then attracted to the surfaces of the micelles by an electrostatic force. For example, the silica and calcium oxide materials assemble between and around ordered surfactant micelles, possibly due to the matching of charge density at the interfaces of the inorganic materials and the surfactants (see, for example, Kresge et al., Nature, Vol. 359, pages 701 to 712, 1992 and Huo et al., Nature, Vol. 368, pages 317 to 321, 1994).

The hydrolysis reaction of step (i) of the method of the present invention may be conducted at any suitable pH. The pH that is suitable will depend on the nature of the structure-directing agent used and will be selected so as to aid the formation of a micelle structure. For example, when the structure-directing agent is a cationic surfactant, step (i) typically is conducted at a basic pH (such as a pH in the range of from 8 to 10, particularly a pH of about 8). When the structure-directing agent is a nonionic surfactant, step (i) typically is conducted at an acidic pH (such as a pH of less than 2, particularity of less than 1).

Step (i) is conveniently carried out at a temperature in the range of, for example, from 20 to 40°C. conveniently at or near 25°C.

It is preferred in step (i) to first dissolve the calcium salt and the structure-directing agent In the aqueous solvent and to then add the tetra(alkyl)silicate to the solution.

Step (i) is conducted in the absence of phosphate ions, so as to produce a calcium oxide-silica composite biomaterial that includes less than 0.5% by weight of phosphate ions. As discussed above, it is believed that this is advantageous because it minimises the formation (and precipitation) of calcium phosphate in the pores of the biomaterial, which in turn aids the formation of hydroxylapatite at the outer surface of the biomaterial.

As the skilled person would appreciate, the step (ii) of isolating a solid from the sol that is formed in step (i) may be conducted by any suitable method or means. For example, the solid may be isolated by simply filtering off the aqueous solvent from the sol. Alternatively, the solid may be isolated by allowing the aqueous solvent to evaporate from the sol.

In step (ii), it is preferred to include the step of washing the isolated solid with water so as to remove any free ions from the solid before step (iii) is conducted. This is especially preferred when the solid is isolated by simply filtering off the aqueous solvent from the sol.

In step (iii) of the method of the present invention, the structure-directing agent is removed from the solid for example by thermal decomposition. In this way, the structure-directing agent is believed to direct the formation of a three-dimensional ordered pore architecture and to direct the formation of specifically sized pores.

It is believed that there is a linear relationship between the unit cell parameter (i.e. the pore size plus pore wall thickness) and the size of the structure-directing agent, for example the number of carbon atoms in the surfactant chain, in acidic and basic media respectively. Typically, the longer the carbon chain of the surfactant then the larger the pore size obtained.

Typically, in step (iii) the calcium salt is converted to calcium oxide (for example, when the calcium salt added in step (i) is calcium nitrate, it is converted to calcium oxide and nitrogen dioxide). The removal of the structure-directing agent provides an ordered three-dimensional pore structure.

As the skilled person would appreciate, the calcination step (iii) may be conducted at any suitable temperature. Suitable temperatures for the calcination step (iii) are those at which the structure-directing agent is thermally decomposed and the calcium salt is substantially converted to calcium oxide. Typically all of the calcium salt is converted to calcium oxide in the calcination step (iii).

As the skilled person would appreciate, the preferred calcination temperature varies according to the particular structure-directing agent used. Typically, the calcination step (iii) is conducted at a temperature in the range of, for example, from 500 to 800°C, particularly of from 500 to 700°C, even more particularly of from 500 to 600°C, for example about 550°C. At these temperatures, the calcium oxide-silica composite biomaterial typically is formed as a powder. It does not form in a glass state or as a monolith (i.e. a single block of material). However, as the skilled person would appreciate, alternative calcination temperatures can be selected if it is desired to form a glass or a monolith.

According to the present invention, there is also provided a calcium oxide-silica composite biomaterial obtainable by a method as defined above. There is also provided a calcium oxide-silica composite biomaterial obtained by a method as defined above.

### Uses

According to present invention, there is also provided the use of a calcium oxide-silica composite biomaterial as herein defined for inducing the formation of hydroxylapatite. The hydroxylapatite typically is formed on the surface of the calcium oxide-silica composite biomaterial.

In order to induce the formation of hydroxylapatite, it is believed that the calcium oxide-silica composite biomaterial of the present invention should be contacted with phosphate ions, for example with an aqueous solution containing phosphate ions at a minimum concentration of about 5 mM. Suitable solutions include, for example, phosphate buffer solution, artificial saliva, simulated body fluid and real human or animal saliva. Upon contact with the solution, the calcium oxide dissolves so as to release calcium ions into solution. The calcium ions and phosphate ions allow the system to reach hydroxylapatite supersaturation level (and hydroxylapatite formation) in a short period of time.

The composition of simulated body fluid in 1 litre solution is:

| Reagent | Amount (g) |
|---|---|
| NaCl | 8.03618 |
| NaHCO₃ | 0.350 |
| KCI | 0.224 |
| K₂HPO₄·3HO₂ | 0.2303 |
| MgCl₂·6HO₂ | 0.31122 |
| HCl (1 mol/L) | 40 ml |

| | |
|---|---|
| CaCl₂ | 0.383476 |
| Na₂SO₄ | 0.0717 |
| (CH₂OH)₃CNH₂ | 0.069138 |

In artificial saliva solution, the calcium ion concentration is 0.9 mM and the phosphate ion concentration is 7mM and the rest of the compositions are the same as simulated body fluid in the present study.

The calcium oxide-silica composite biomaterials of the present invention are believed to be advantageous because, in use, they induce hydroxylapatite formation by an efficient and fast mechanism. As discussed above, it is believed that the calcium oxide-silica composite biomaterials of the present invention effectively release calcium ions into solution and can control the crystallisation site. It is believed that the calcium oxide-silica composite biomaterials of the present invention direct the formation of hydroxylapatite at the surface of the biomaterials. In most cases of tissue regeneration, the formation of hydroxyapatite at the surface of the biomaterial is the prerequisite step for the subsequent reactions of that bond the tissue to the cells.

Typically, the calcium oxide-silica composite biomaterials of the present invention can induce the formation of hydroxylapatite in a faster time than the biomaterials of the prior art. For example, the biomaterials of the present invention typically can induce the formation of hydroxylapatite in a time period of less than 4 hours. After 24 hours, typically about 40% by weight of the calcium oxide-silica composite biomaterials will have been transformed into crystalline hydroxylapatite.

According to another aspect of the present invention, there is provided a method of forming hydroxylapatite, the method comprising the step of contacting the calcium oxide-silica composite biomaterial as herein defined with phosphate ions at a pH in the range of from 5 to 10, particularly of from 6.8 to 7.2, for example at a pH of about 7. The preferred pH depends on the particular application, For example, for the formation of hydroxylapatite *in vivo,* the preferred pH is in the range of from 6.8 to 7.2, for example about 7.

Typically, at least 98% by weight of the hydroxylapatite forms on the surface of the biomaterial. This can be confirmed by X-ray diffraction, scanning electron microscopy (SEM) and transmission electron microscopy (TEM). The hydroxylapatite formed is preferably in a crystalline state.

The phosphate ions may, for example, be provided in solution, for example in a phosphate buffer solution, in artificial saliva, in simulated body fluid or in real human or animal saliva.

According to another aspect of the present invention, there is provided the use of a calcium oxide-silica composite biomaterial as herein defined in tissue regeneration. In this aspect, the tissue may be soft or hard tissue. By the term "regeneration", we include restoration and remineralisation processes.

According to another aspect of the present invention, there is provided the use of a calcium oxide-silica composite biomaterial as herein defined in tooth and/or bone regeneration. For example, we include the restoration of tooth dentin and bone, as well as the remineralisation of tooth dentin and tooth enamel. The remineralisation is intended to form new tooth or bone tissue, but not necessarily to restore the old tooth or bone to its original state. In the remineralisation process, hydroxylapatite is deposited onto the substrate (such as bone or tooth) and incorporated into the substrate at any location where there is a crack or lesion. Etching of the substrate surface before the hydroxylapatite is deposited may help to incorporate the hydroxylapatite internally into the substrate.

According to another aspect of the present invention, there is provided the use of a calcium oxide-silica composite biomaterial as herein defined for whitening a tooth.

According to another aspect of the present invention, there is provided the use of a calcium oxide-silica composite biomaterial as herein defined for treating and/or preventing tooth hypersensitivity.

The calcium oxide-silica composite biomaterials of the present invention may be used alone but will generally be administered in the form of a composition in which the calcium oxide-silica composite biomaterial is in association with an acceptable carrier. Typically, the composition will take the form of a paste, gel or cement. The composition may also take the form of a powder, which may be applied to a substrate on a strip (for example Bondi strip) or as a spray (for example in combination with an inactive powder, such as silica or calcium carbonate powder).

Thus, according to another aspect of the present invention, there is provided a composition comprising a calcium oxide-silica composite biomaterial as herein defined.

According to another aspect of the present invention, there is provided a tissue regeneration composition comprising a calcium oxide-silica composite biomaterial as herein defined.

According to another aspect of the present invention, there is provided a bone regeneration composition comprising a calcium oxide-silica composite biomaterial as herein defined.

According to another aspect of the present invention, there is provided a tooth regeneration composition comprising a calcium oxide-silica composite biomaterial as herein defined.

According to another aspect of the present invention, there is provided a tooth whitening composition comprising a calcium oxide-silica composite biomaterial as herein defined.

According to yet another aspect of the present invention, there is provided a composition for treating and/or preventing tooth hypersensitivity, which composition comprises a calcium oxide-silica composite biomaterial as herein defined.

The compositions of the present invention may be in any suitable form, such as in the form of a cement, a paste or a gel. The compositions of the present invention may comprise any suitable carrier, such as a polymer gel carrier.

According to another aspect of the present invention, there is provided a toothpaste comprising a calcium oxide-silica composite biomaterial as herein defined. The toothpaste may comprise any suitable additional ingredients, such as ingredients selected from silica, calcium carbonate, surfactant, perfume and water, and mixtures thereof.

The amount of calcium oxide-silica composite biomaterial that is combined with the carrier(s) will necessarily vary depending upon nature of the material to which and the area to which it is to be applied and on the particular route of administration. A suitable ratio of calcium oxide-silica composite biomaterial to carrier is, for example, in the range of from 1:100 to 1:1.

The present invention further provides a method for the preparation of composition of the invention, which method comprises the step of combining a calcium oxide-silica composite biomaterial as herein defined with an acceptable carrier.

The present invention also provides the use of a calcium oxide-silica composite biomaterial as herein defined for inducing the formation of hydroxylcarbonate apatite. Typically, the hydroxylcarbonate apatite is formed on the surface of the calcium oxide-silica composite biomaterial.

In order to induce the formation of hydroxylcarbonate apatite, it is believed that the calcium oxide-silica composite biomaterial of the present invention should be contacted with phosphate ions in the presence of carbon dioxide. Suitable sources of phosphate ions are discussed above.

According to another aspect of the present invention, there is provided a method of forming hydroxylcarbonate apatite, the method comprising the step of contacting the calcium oxide-silica composite biomaterial as herein defined with phosphate ions at a pH in the range of from 5 to 10 (particularly of from 6.8 to 7.2, for example at a pH of about 7) in the presence of carbon dioxide. The formation of hydroxylcarbonate apatite is desirable in the regeneration of bone tissue.

The present invention will now be described further with reference to the following examples which are illustrative only and non-limiting.

In the examples, the average pore size of the biomaterials was measured by BET nitrogen sorption. The instrument used was a Micromeritics Tristar 3000 analyzer (from Micromeritics GmbH, Mönchengladbach, Germany). The nitrogen adsorption measurements were performed at 77K in nitrogen gas using 0.1 g biomaterial as a powder. The powder was preheated at 200°C for 2 hours before testing to eliminate water.

The BET specific surface area was measured using a TriStar 3000 Analyzer, which uses physical adsorption and capillary condensation principles to obtain information about the surface area and porosity of a solid material. A sample contained in an evacuated sample tube was cooled to cryogenic temperature and then exposed to analysis gas at a series of precisely controlled pressures. With each incremental pressure increase, the number of gas molecules adsorbed on the surface increases. The equilibrated pressure (P) was compared to the saturation pressure (Po) and their relative pressure ratio (P/Po) was recorded along with the quantity of gas adsorbed by the sample at each equilibrated pressure. As adsorption proceeds, the thickness of the adsorbed film increases. Any micropores in the surface are filled first, then the free surface becomes completely covered, and finally the larger pores are filled by capillary condensation. The process may continue to the point of bulk condensation of the analysis gas. Then, the desorption process may begin in which pressure systematically is reduced resulting in liberation of the adsorbed molecules. As with the adsorption process, the changing quantity of gas on the solid surface at each decreasing equilibrium pressure is quantified. These two sets of data describe the adsorption and desorption isotherms. Analysis of the shape of the isotherms yields information about the surface and internal pore characteristics of the material.

The instrument used for the X-ray diffraction (XRD) measurements was a Rigaku, D/MAX, 2500, Japan. This instrument utilizes the monochromatic X-rays to determine the interplanar-spacings (d-spacing) of the biomaterials. Samples were analysed as powders with grains in random orientations to insure that all crystallographic directions are "sampled" by the beam.

The instrument used for the scanning electronic microscopy (SEM) was a JEOL, JSM-6700F Filed emission (made by JEOL, Japan).

The instrument used for the high resolution- transmission electronic microscopy (HRTEM) measurements was a JEOL, 2010F (made by JEOL, Japan). The 2010F is an energy filtering, field-emission analytic TEM/STEM. It operates at 200kV and uses a Schottky field emitter.

The instrument used for Raman spectroscopy measurements was a LabRam-1B, HORIBA Jobin Yvon Ltd, UK.

### BRIEF DESCRIPTION OF THE DRAWINGS

The present invention will now be explained in more detail by way of the following Example and with reference to the accompanying drawings in which:-
Figure 1 shows pore size distributions for a material according to the present invention and a comparative material;
Figure 2 shows a small angle X-ray diffraction pattern for a material according to the present invention;
Figure 3 shows SEM images of calcinated CaO-SiO₂ composite materials before and after incubation;
Figure 4 shows X-ray diffraction patterns for another material according to the present invention and of a comparative example;
Figure 5 shows SEM images of a calcinated CaO-SiO₂ composite material according to the present invention and of a comparative example (scale bar 1 µm);
Figure 6 shows SEM images of the materials of Example 3;
Figure 7 shows SEM images of the materials of Example 4 (scale bar 1 µm);
Figure 8 shows SEM images of a tooth covered with a material according to the present invention, before and after incubation;
Figure 9 shows HRTEM images for the material of Example 1, after incubation;
Figure 10 shows a larger area HRTEM image of the sample depicted in Figure 9;
Figures 11 - 13 show HRTEM images obtained from the material of Comparative Example 1; and
Figures 14 shows a respective Raman spectra of an etched tooth sample before and after treatment with the material of Example 1.

### EXAMPLES

### Example 1

Cetyltrimethylammonium bromide (CTAB) powder (1.3 g) was dissolved in a mixture of deionised water (25 g) and ethanol (30 g). The weight ratio of CTAB to liquid is 0.02. The solution was stirred at 25°C for 10 minutes, after which time the CTAB had dissolved and the solution appeared clear. Ca(NO₃)₂ (2.36 g) was then added to the CTAB solution. Ammonia solution (25%, 1.6 g) was then added to obtain a pH of about 8. The solution was still transparent, which indicated that Ca(OH)₂ had not formed yet and calcium was in free ion form. Then liquid TEOS (6 g) was added drop-wise to the basic solution, with violent stirring. After 1 hour, the clear solution became cloudy. This showed that TEOS had started to hydrolyse. The stirring was continued for 24 hours at 25°C, until most of the solution had become a sol.

The hydrolysis product of the sol was then vacuum filtered and washed twice with deionised water (to remove the free ions in the solution). Then the solid collected by filtration was dried at 100°C for 12 hours. Finally the dried solid was calcinated at 550°C for 5 hours and allowed to cool in the oven to bum off the CTAB so as to form the pores. At this temperature, calcium nitride was also decomposed into CaO and NO₂. The SiO₂-CaO composite did not form a glass state and was not a monolith. Instead, the composite was a powder.

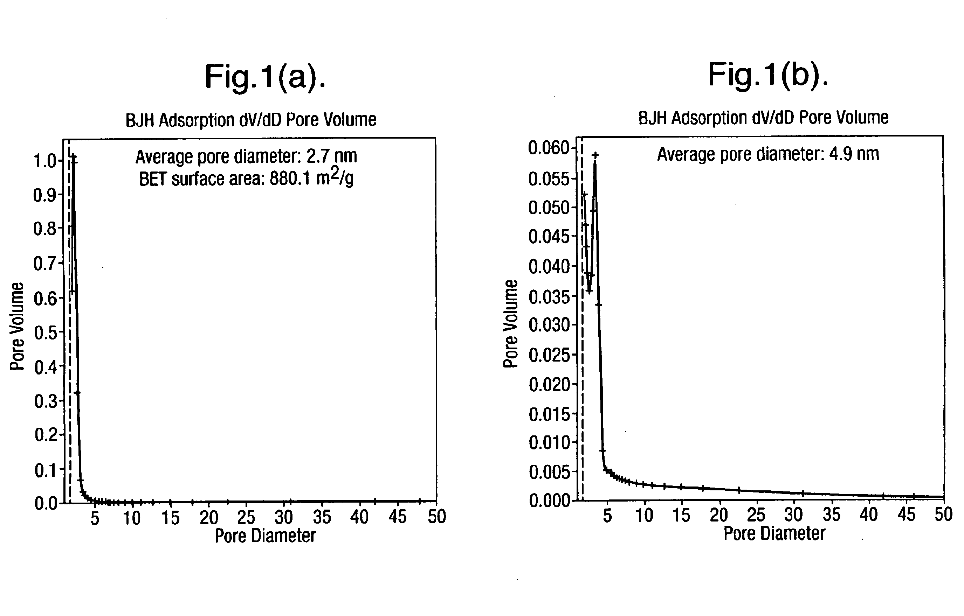

Figure 1 (a) shows the pore size distribution of the material measured by BET nitrogen sorption. The average pore size was found to be 2.7 nm. The measured BET specific surface area was 880.1 m²/g.

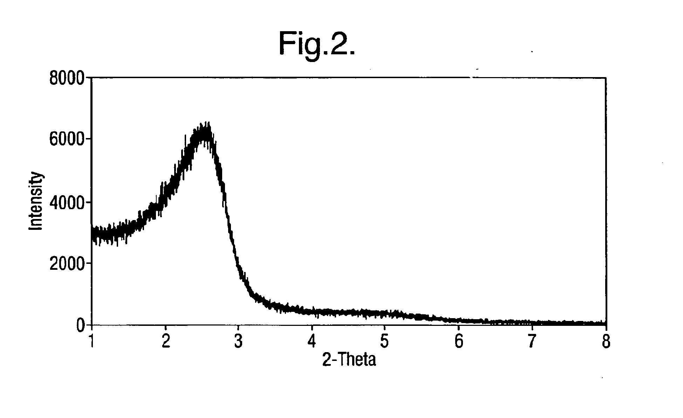

Figure 2 shows the small angle X-ray diffraction pattern of the material. A rising peak is shown at 2.5°, which is indicative of mesopore formation as well as the ordering of the pores.

The powder was then ground in a pestle and the ground powder (0.2 g) was poured into phosphate buffer solution (PBS, 30 ml) in a Pyrex glass bottle. PBS was prepared by dissolving Na₂HPO₄ (3.533 g) and KH₂PO₄ (3.387 g) in deionised water (1 litre) at a pH of 6.8. Three different concentrations of PBS were used to evaluate the effect of the phosphate concentration. These were (1) normal PBS prepared with a phosphate concentration of 24.9 mM, (2) 5 times dilute, called PBS-Dilute 5, and (3) 10 times dilute, called PBS- Dilute 10. The mouth of the glass bottle was sealed. Then plastic was wrapped around the glass mouth and the sealed bottle (containing the powder sample and the PBS solution) was placed in a water bath incubator (Model: DKZ-Z; Company name: Shanghai Fuma Experimental Equipment Co. Ltd., Shanghai, China) with a gentle shaking at a temperature of 37°C (± 0.1 °C). Samples (2 to 3 ml) were removed after 1 hour, 4 hours, 8 hours, 1 day and 12 days. These samples were quickly transferred into a refrigerator for freezing to keep them in their original state until they were characterised by X-ray diffraction, SEM and TEM.

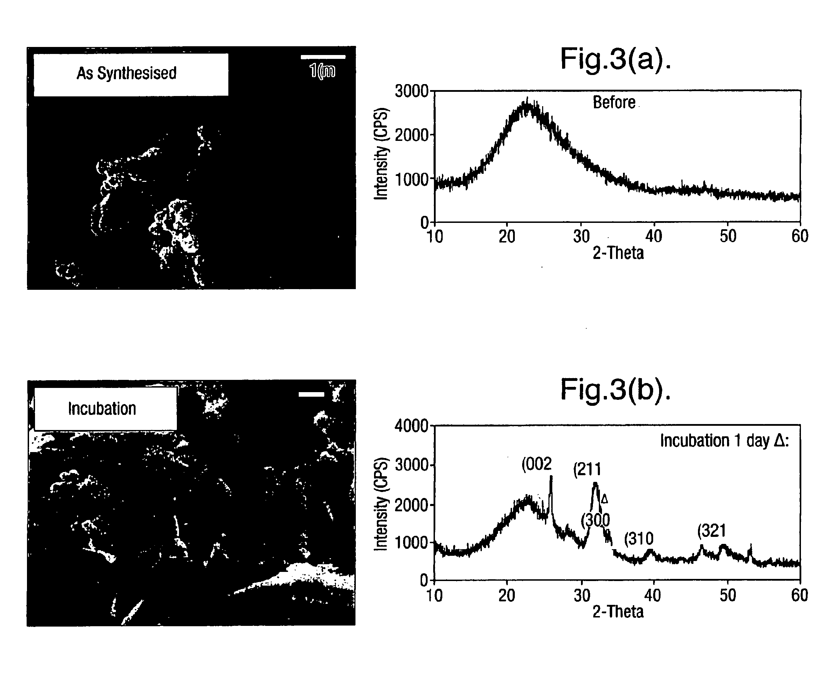

Figure 3 shows SEM images of the calcinated CaO-SiO₂ composite material before (Figure 3 (a)) and after 1 days incubation (Figure 3 (b)). The samples showed that full crystalline hydroxylapatite had formed after 1 day.

### Comparative Example 1

The procedure of Example 1 was repeated using Pluronic F127® instead of CTAB. Pluronic F127® (6 g) was added to deionised water (30 g) with stirring at 60°C. Then HCl (2M, 112 g) was added to the solution. Liquid TEOS (12 g) was added drop-wise to the acidic solution and the resulting solution was stirred vigorously for 24 hours. The solution became cloudy after 12 hours.

Figure 1 (b) shows the pore size distribution of the material measured by BET nitrogen sorption. The average pore size was found to be 4.9 nm. The measured BET specific surface area was 400.1 m²/g.

### Example 2

The calcinated CaO-SiO₂ composite material from Example 1 (0.1 g) was incubated in a phosphate buffer solution (PBS, 25mM, 30 ml) at a pH of 6.8. The incubation temperature was set to 37°C. After incubation for 1 day, the sample was taken out, filtered and dried.

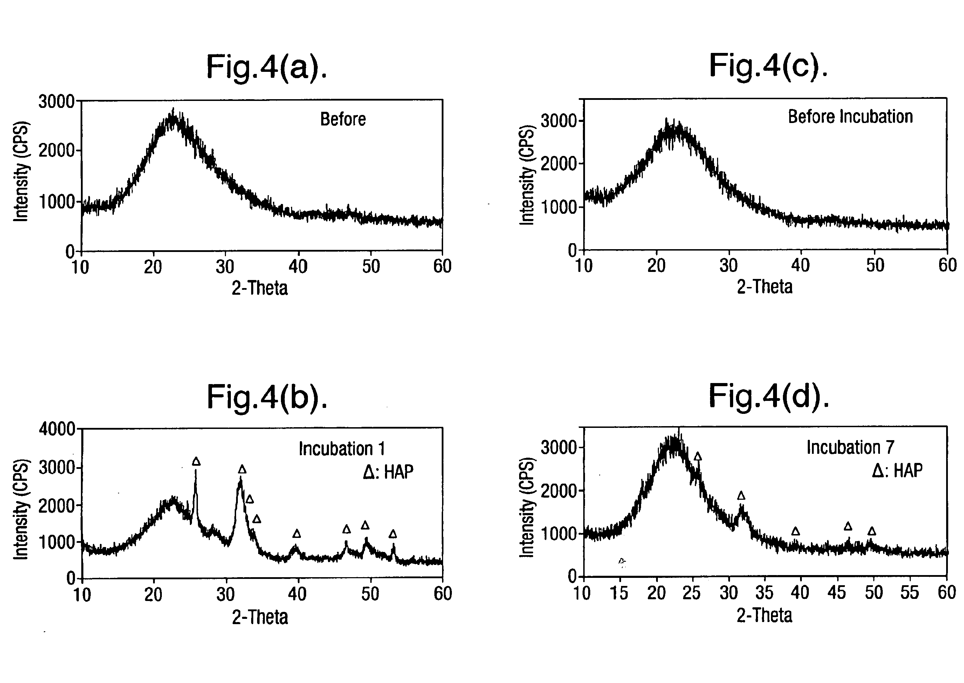

The dried sample was characterised by X-ray diffraction (XRD). The XRD spectrum of this sample is shown in Figures 4 (a) and (b), both before and after incubation for 1 day. Clearly, before incubation, there are no sharp peaks showing that only the amorphous phase existed (Figure 4 (a)). After incubating for 1 day, a full pattern of XRD peaks appeared, which peaks were confirmed as representing hydroxylapatite, by using software produced by Materials Data, Inc (which software can identify phases in a sample, characterize density and lattice constants) indicated as triangle symbols (Figure 4 (b)). All of the peaks shown relate to hydroxylapatite, showing that the hydroxylapatite was the only product of the incubated sample. No other impurity or other types of calcium phosphate were present. The mature pattern of the hydroxylapatite XRD peaks also provided direct information for the fully-grown out new phase of hydroxylapatite.

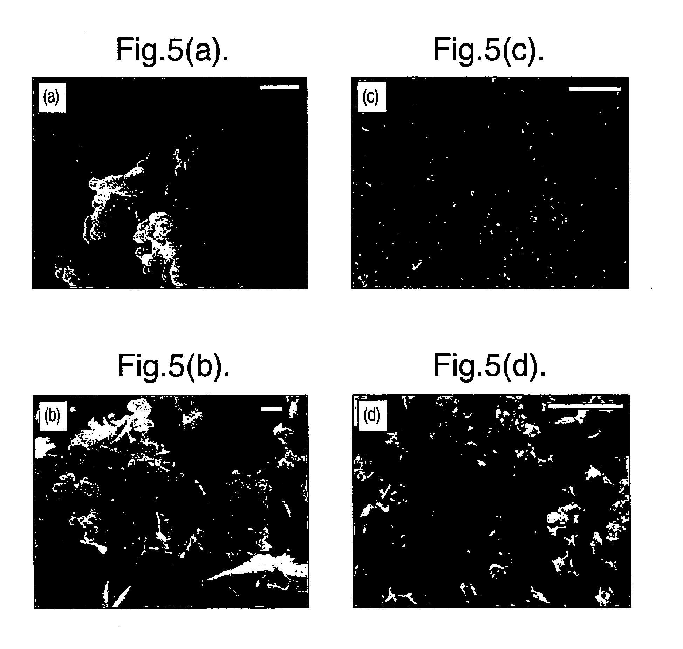

The microscopic morphology of the samples was observed before and after they were incubated, using scanning electronic microscopy. Figure 5 shows SEM images of the calcinated CaO-SiO₂ composite material from Example 1 before (Figure 5 (a)) and after 1 day incubation (Figure 5 (b)). Before incubation, the material was in a spherical shape and had a smooth surface. The spherical diameter of the particles was between 0.2 and 0.5 µm and most of them were aggregated. After incubating in a PBS solution at 37°C for 1 day, full crystalline hydroxylapatite was grown out from the spherical particle substrate. The hydroxylapatite crystals were in a plate-like shape and of a size of between 1 to 10 µm (i.e. much larger than its substrate particle). These platelet hydroxylapatite crystals were covering almost all the substrate surface of calcinated CaO-SiO₂ powder, forming a full but not dense layer of hydroxylapatite.

### Comparative Example 2

Example 2 was repeated using the calcinated CaO-SiO₂ composite material from Comparative Example 1.

The dried sample was characterised by X-ray diffraction (XRD). The XRD spectrum of this sample is shown in Figures 4 (c) and (d), both before and after incubation for 7 days. Clearly, before incubation, there were no sharp peaks showing that only the amorphous phase existed (Figure 4 (c)). After incubating for 7 days, a weak pattern of XRD peaks corresponding to hydroxylapatite appeared (Figure 4 (d)).

The microscopic morphology of the calcinated CaO-SiO₂ composite material from Comparative Example 1 was observed before and after they were incubated, using scanning electronic microscopy (SEM). Figure 5 shows SEM images of the calcinated CaO-SiO₂ composite material before (Figure 5 (c)) and after 7 days incubation (Figure 5 (d)). Before incubation, the calcinated CaO-SiO₂ material from Comparative Example 1 was irregular and had a smooth surface. After incubating these particles into PBS solution at 37°C for 7 days, star-like hydroxylapatite crystallites were grown out of the original smooth substrate, as shown in Figure 5 (d). The average size of the hydroxylapatite crystal plates was less than 0.5 µm, much smaller than that from the calcinated CaO-SiO₂ composite material from Example 1.

### Discussion of Example 2 and Comparative Example 2

A comparison of Example 2 and Comparative Example 2 shows that the calcinated CaO-SiO₂ material of Example 1 produces hydroxylapatite in a more mature crystalline form (because the hydroxylapatite crystallite produced are much bigger in size) and in larger quantities than the calcinated CaO-SiO₂ composite material of Comparative Example 1. Surprisingly, the time it took to form hydroxylapatite was much shorter for the calcinated CaO-SiO₂ composite material of Example 1 than for the calcinated CaO-SiO₂ composite material of Comparative Example 1. The calcinated CaO-SiO₂ composite materials of Example 1 and of Comparative Example 1 have substantially the same chemical composition and differ only in their pore size, as discussed above.

In summary, XRD and SEM results have demonstrated that calcinated CaO-SiO₂ composite material from Example 1 has much higher capability to induce hydroxylapatite formation than the calcinated CaO-SiO₂ composite material from Comparative Example 1. The former can produce more hydroxylapatite crystallites in shorter incubation time.

### Example 3

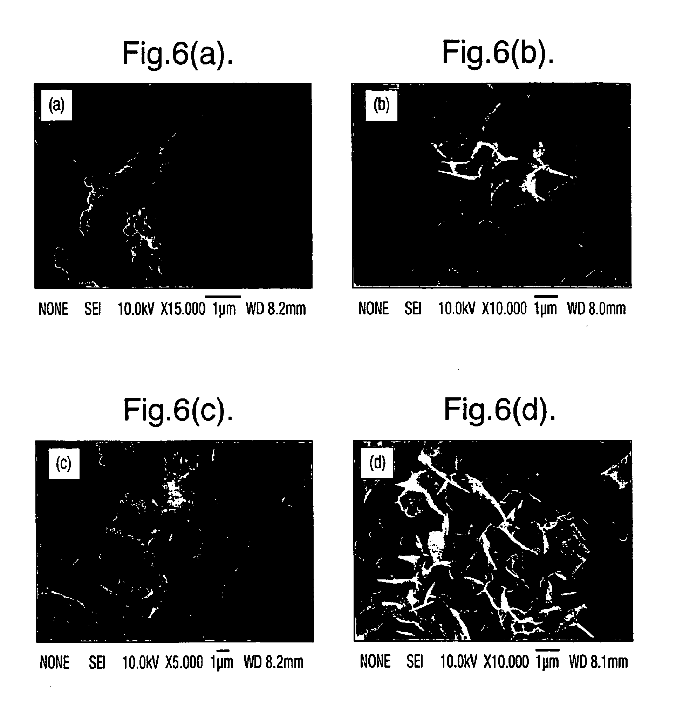

A much shorter incubation time was tested for the sample of the calcinated CaO-SiO₂ composite material from Example 1. The procedure of Example 2 was repeated, except that samples were removed after incubation for 1 hour, 4 hours and 8 hours. Then scanning electronic microscopy (SEM) was used to observe the morphology change. The results are shown in Figure 6, in which (a) is the sample before incubation and (b), (c), (d) are the samples after incubation for 1 hour, 4 hours and 8 hours respectively. Before incubation, the sample had a smooth surface and a spherical shape (see Figure 6 (a)). After incubation for 1 hour, fully crystalline hydroxylapatite had grown with a platelet-like shape (see Figure 6 (b)). After incubation for 4 hours, the HA plates had grown larger, gradually covering all of the substrate (see Figure 6 (c)). After incubation for 8 hours, the HA plates had formed a full rounded pattern (see Figure 6 (d)).

### Example 4

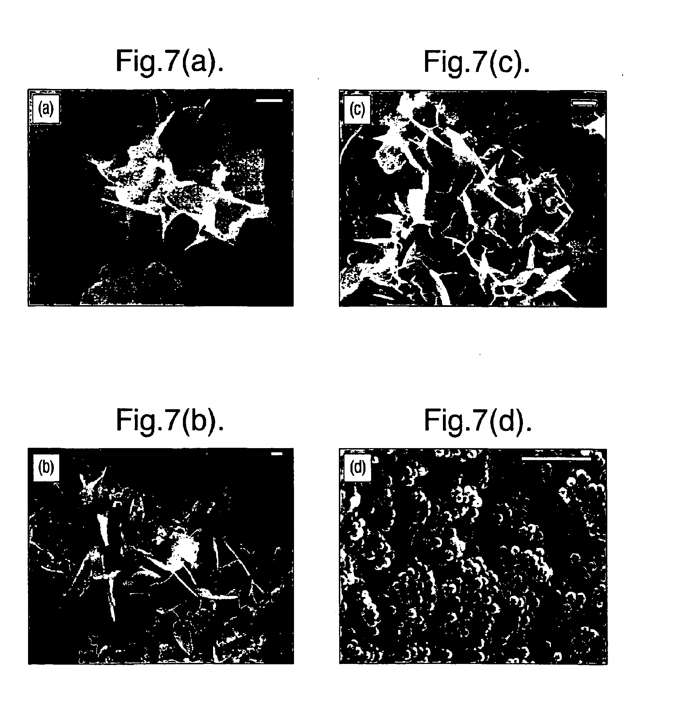

The procedure of Example 3 was repeated, except that a control sample was included. The control sample was prepared in the same way as the calcinated CaO-SiO₂ composite material from Example 1 except that no structure-directing agent (i.e. no CTAB) was included. Figures 7 (a) to (c) show SEM images for the calcinated CaO-SiO₂ composite material from Example 1 after 1, 4 and 8 hour incubation times. Figure 7 (d) shows the SEM image for the control sample after incubation for 24 hours. Even after 1 hour incubation time, the calcinated CaO-SiO₂ composite material from Example 1 formed fully crystallized hydroxylapatite plates (Figure 7 (a)). The plates were relatively small (around 1 µm in its edge dimension) and grew out into a blossom-like pattern, starting from one location and forming a large cluster of greater than 5 µm in it lateral dimension. After 4 hours incubation, the calcinated CaO-SiO₂ composite material from Example 1 produced more hydroxylapatite flower-like clusters in higher density (Figure 7 (b)). After 8 hours incubation time, the calcinated CaO-SiO₂ composite material from Example 1 produced a fully-fledged, spherical, peony-flower like hydroxylapatite crystal, of about 10 µm in diameter (Figure 7 (c)). In comparison, the SEM image for the control sample after incubation for 24 hours shows that not a single crystal plate formed (Figure 7 (d)). Very smooth spherical particles remained and no new phase had formed.

### Example 5

The calcinated CaO-SiO₂ composite material from Example 1 was applied in the form of a gel to treat a damaged human tooth.

The gel was prepared by mixing the calcinated CaO-SiO₂ composite material from Example 1 (0.2 g) with a carrier material (0.5 g) and a phosphate buffer solution (pH 7, 50 mM) at a temperature in the range of from 60 to 80°C with quick stirring. The solution was then cooled to room temperature and a white gel formed.

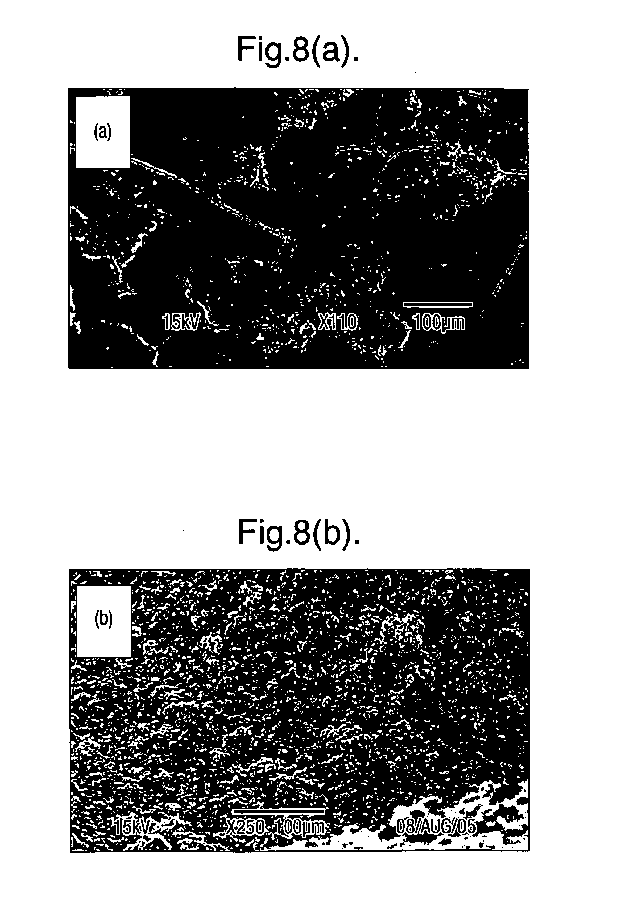

The gel was then applied to a human tooth and incubated for 1 day at 37°C in a simulated oral fluid (having a calcium concentration of 0.9 mM and a phosphate concentration of 7 mM). The gel-coated tooth was then washed three times with distilled water and observed using SEM. Figure 8 shows the SEM results. Before incubation, there are many micro size cracks on the surface of the tooth enamel (see Figure 8 (a)). After incubation with the gel, a uniform covering layer of hydroxylapatite was coated on the cracked tooth surface (see Figure 8 (b)).

### Example 6

The nucleation behaviour of hydroxylapatite formed from the calcinated CaO-SiO₂ composite materials from Example 1 and Comparative Example 1 was studied by high resolution- transmission electronic microscopy (HRTEM).

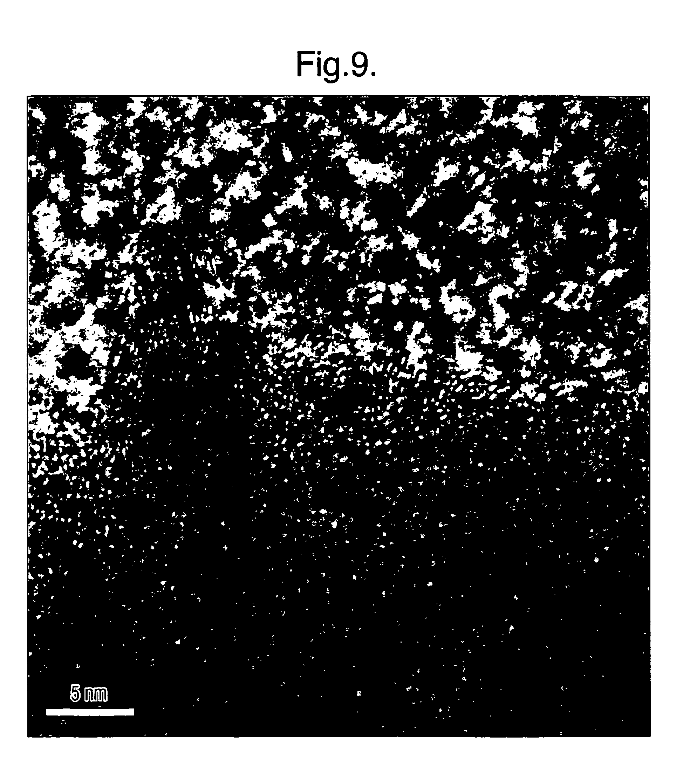

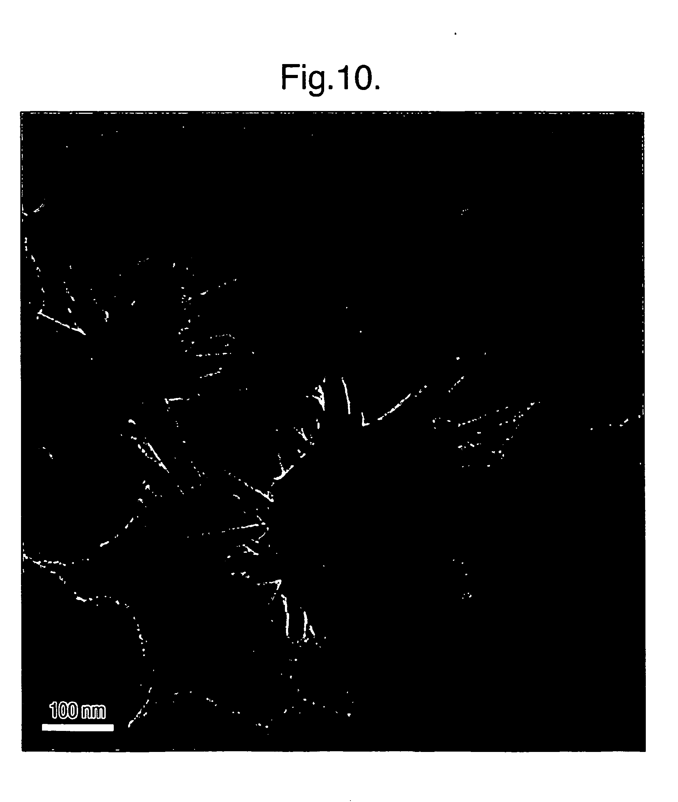

Figure 9 shows HRTEM images for the calcinated CaO-SiO₂ composite material from Example 1 after incubation for 1 hour as described in Example 2. An embryonic crystalline hydroxylapatite starts to grow in a plane (112) from the edge of amorphous calcinated CaO-SiO₂ composite material from Example 1 (see Figure 9). The hydroxylapatite embryo is very small, marked by a white rectangle. Figure 10 shows a larger area view of the new hydroxylapatite phase formed from the calcinated CaO-SiO₂ composite material from Example 1. More needle-like hydroxylapatite crystallites are gown out from the edge of the spherical shape substrate. Those needles will further grow into a larger plate-like shape, for example as shown in Figure 7. During all the time that the calcinated CaO-SiO₂ composite material from Example 1 was observed by HRTEM, no nucleated hydroxylapatite crystallite was found inside the matrix of the material. This shows that the pores in this material suppress the nucleation. Without wishing to be bound by any theory, is believed that this is due to size of the pores.

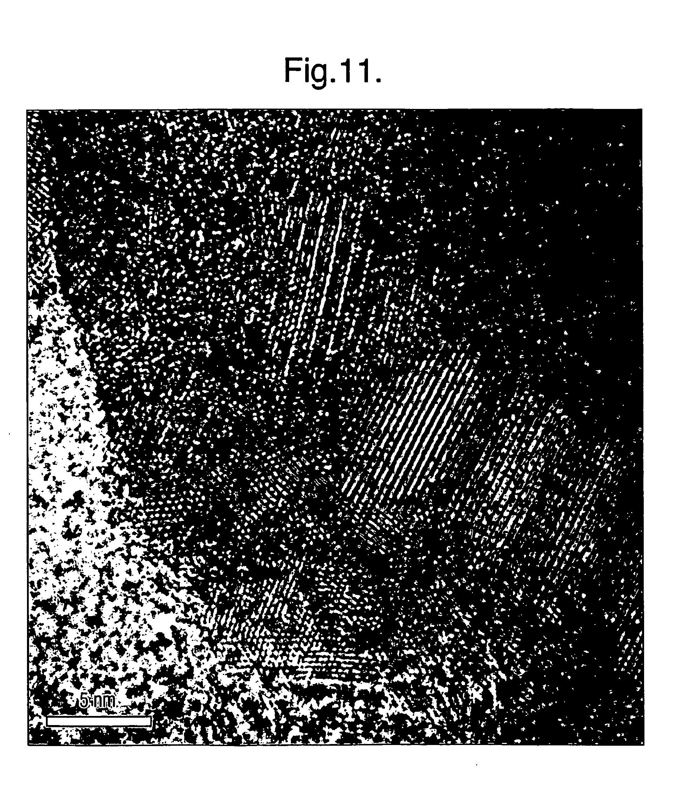

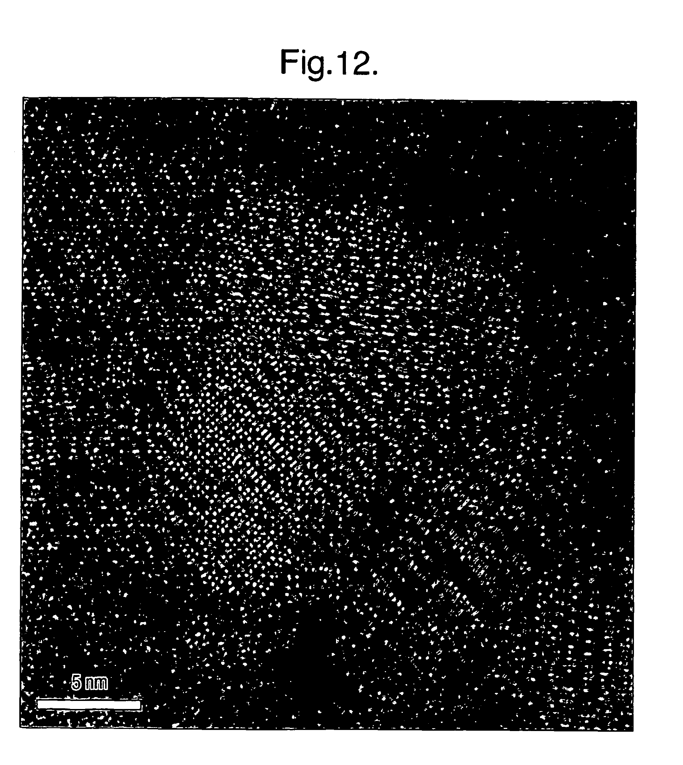

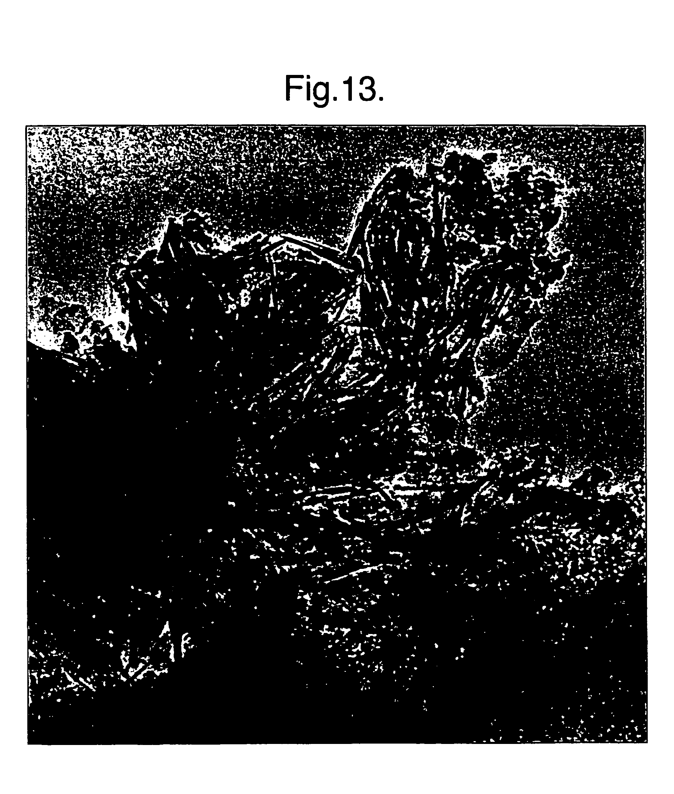

In comparison, the calcinated CaO-SiO₂ composite material from Comparative Example 1 was also observed by HRTEM following incubation for 7 days, as shown in Figures 11 to 13. Nanosize hydroxylapatite crystals were found to start the nucleation within the amorphous calcinated CaO-SiO₂ composite material from Comparative Example 1 (see Figure 11). The lattice constants were measured as 8.160 angstrom in the upper domain, representing hydroxylapatite (100) plane and 3.525 angstrom in the lower domain, representing hydroxylapatite (201) plane. The diameter of the newly formed hydroxylapatite domain is about 5 nm (comparable to the BET measured average diameter of 4.9 nm in calcinated CaO-SiO₂ composite material from Comparative Example 1). So, the hydroxylapatite is believed to seed its nuclei inside the pores and then the nuclei grow until they are of a size that fills the pores/channels between adjacent pores, indicated by the small regions/domains of hydroxylapatite. Figure 12 further shows that a well crystalline hydroxylapatite cluster was formed away from the surface of the parental matrix in a size of about 5 nm. However, another much larger hydroxylapatite crystallite was formed close to the surface of amorphous matrix, with its diameter larger than 5 nm. It is believed that this may be because the crystallite is located closer to the edge of the calcinated CaO-SiO₂ composite material from Comparative Example 1 and so it is not constrained by the pore size. A larger area view of crystalline hydroxylapatite from calcinated CaO-SiO₂ composite material from Comparative Example 1 is shown in Figure 13. Both needle and plate-like hydroxylapatite crystals are shown adjacent to the amorphous area. This shows that the hydroxylapatite is able to nucleate inside the pores of the calcinated CaO-SiO₂ composite material from Comparative Example 1.

### Example 7

A human tooth was etched with 37wt% phosphate acid *in vitro.* The tooth was immersed in the acid solution for 60 seconds and then rinsed thoroughly to wash off the residue of phosphate acid.

The tooth was treated with a gel comprising the calcinated CaO-SiO₂ composite material from Example 1 (0.15 g), water (10 g) and gel carrier (0.15 g) and then incubated for one week at 37°C in a phosphate buffer solution of concentration of 50 mM.

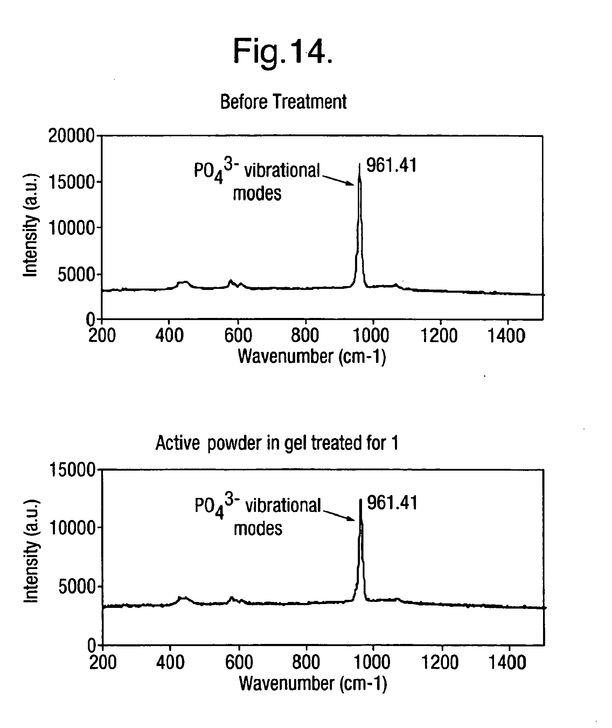

Figures 14 (a) and (b) show SEM images of the tooth sample. Figure 14 (a) shows the tooth after the acid etching and Figure 14 (b) shows the acid etched tooth after treatment with the gel. It is clear from Figure 14 (b) that a thin layer of new repair coating was formed on the acid etched tooth sample.

Raman spectroscopy was used to detect the surface chemistry. Figure 14 (c) shows the Raman spectrum for the acid etched tooth without gel treatment and Figure 14 (d) shows the Raman spectrum for the tooth after treatment with the gel. The Raman spectra shown in Figures 14 (c) and (d) are identical, which means that they have identical surface chemical composition. This shows that the repairing layer formed by the gel is hydroxylapatite, the same material as the tooth enamel.

## Claims

1. A calcium oxide-silica composite biomaterial either in amorphous state or crystalline state having an average pore size, as determined by the BET method, in the range of from 0.8 to 4 nm, wherein the calcium oxide-silica content of the biomaterial is at least 80 wt %. and wherein the molar ratio of calcium oxide to silica is at least 0.1, and wherein the biomaterial includes less than 0.5% by weight of phosphate ions and wherein the pores of the biomaterial have an ordered arrangement.

2. A calcium oxide-silica composite biomaterial according to claim 1, wherein the average pore size is in the range of from 1 to 2.7 nm,

3. A calcium oxide-silica composite biomaterial according to any of the preceding claim that is in the form of a powder.

4. A method for preparing a calcium oxide-silica composite biomaterial according to claim 1, the method comprising the steps of:
(i) combining, in solution, a calcium salt, an organic or inorganic silica precursor such as a silicate or a tetra(alkyl)silicate and a structure-directing agent in the presence of an aqueous solvent whereby hydrolysis of the tetra(alkyl)silicate occurs, leading to the formation of a sol;
(ii) isolating a solid from the sol; and
(iii) calcinating the isolated solid
and wherein the structure-directing agent is selected from a surfactant of the formula CₙH₂ₙ₊₁N(CH₃)₃X, wherein X is bromo or chloro and n is 8, 10, 12, 14 or 16, a surfactant of the formula CₘH₂ₘ₊₁NH₂, wherein m is 8, 10, 12, 14, 16 or 18, a nanionic block copolymer surfactant ot the Formula EO₁₀₀ PO₃₉ EO₁₀₀, wherein EO represents polyl(ethylene)oxide and PO represents poly(propylene)oxide, and a nonionic star copolymer surfactant of the formula (EO₁₁₃ PO₂₂)₂ N(CH₂)₂ N(PO₂₂ EO₁₁₃)₂, wherein EO represents poly(ethylene)oxide and PO represents poly(propylene)oxide," and mixtures thereof.

5. A method according to daim 4, wherein the tetra(alky)silicate is selected from tetramethyl orthosilicate and tetraethyl orthosilicate.

6. A method according to claim 5, wherein the silica precursor is an organic precursor which is a tetra(alky)silicate in the form of tetraethyl orthosilicate.

7. A method according to any one or more of claims 4 to 6, wherein the calcium salt is selected from calcium nitrate, calcium fluoride and calcium chloride.

8. A method according to claim 5, wherein the structure-directing agent is selected from cetyttrimethylammonium bromide, a nonionic block copolymer surfactant of the Formula EO₁₀₀ PO₃₉ EO₁₀₀, EO represents poly(ethylene)oxide and PO represents poly(propylen)oxide, a nonionic star copolymer surfactant of the Formula (EO₁₁₃ PO₂₂)₂ N(CH₂)₂ N(PO₂₂ EO₁₁₃)₂₉ wherein EO represents poly(ethylene)oxide and PO represents poly(propylene)oxide and dodecylamine.

9. A method according to any one or more of claims 4 to 8, wherein the aqueous solvent comprises water and a C₁-C₄ alcohol.

10. A method according to any one or more of claims 4 to 9, wherein in step (ii) the solid is isolated by filtration.

11. A method according to any one or more of claims 4 to 9, wherein in step (ii) the solid is isolated by evaporation.

12. A method according to any one or more of claims 4 to 11, wherein the calcination in step (iii) is conducted at a temperature in the range of from 500 to 800°C.

13. A method of forming hydroxylapatite, the method comprising the step of contacting the calcium oxide-silica composite biomaterial according to any one or more of claims 1 to 3 with phosphate ions at a pH in the range of from 5 to 10.

14. A calcium oxide-silica composite biomaterial according to any one or more of claims 1 to 3 for use in tissue regeneration.

15. A calcium oxide-silica composite biomaterial according to any one or more of claims 1 to 3 for use in tooth and/or bone regeneration.

16. A calcium oxide-silica composite biomaterial according to any one or more of claims 1 to 3 for use in whitening a tooth.

17. A calcium oxide-silica composite biomaterial according to any one or more of claims 1 to 3 or use in treating and/or preventing tooth hypersensitivity,

18. A tissue regeneration composition comprising a calcium oxide-silica composite biomaterial according to any one or more of claims 1 to 3.

19. A bone regeneration composition comprising a calcium oxide-silica composite biomaterial according to any one or more of claims 1 to 3.

20. A tooth regeneration composition comprising a calcium oxide-silica composite biomaferial according to any one or more of claims 1 to 3.

21. A tooth whitening composition comprising a calcium oxide-silica composite 3. biomaterial according to any one or more of claims 1 to 3.

22. A composition for treating and/or preventing tooth hypersensitivity, which composition comprises a calcium, oxide-silica composite biomaterial according to any one or more of claims 1 to 3.

## Patentansprüche

1. Calciumoxid-Siliciumdioxid-Verbundbiomaterial entweder in amorphem Zustand oder in kristallinem Zustand, das eine durchschnittliche Porengröße, wie sie durch die BET-Methode bestimmt wird, im Bereich von 0,8 bis 4 nm hat, wobei der Calciumoxid-Siliciumdioxid-Gehalt des Biomaterials wenigstens 80 Gew.-% ist und wobei das Molverhältnis von Calciumoxid zu Siliciumdioxid wenigstens 0,1 ist und wobei das Biomaterial weniger als 0,5 Gew.-% Phosphationen umfasst und wobei die Poren des Biomaterials eine geordnete Anordnung haben.

2. Calciumoxid-Siliciumdioxid-Verbundbiomaterial gemäß Anspruch 1, wobei die durchschnittliche Porengröße im Bereich von 1 bis 2,7 nm ist.

3. Calciumoxid-Siliciumdioxid-Verbundbiomaterial gemäß einem der vorangehenden Ansprüche, das in der Form eines Pulvers ist.

4. Verfahren zur Herstellung eines Calciumoxid-Siliciumdioxid-Verbundbiomaterials gemäß Anspruch 1, wobei das Verfahren die folgenden Schritte umfasst:
(i) Kombinieren, in Lösung, eines Calciumsalzes, eines organischen oder anorganischen Siliciumdioxid-Vorläufers, zum Beispiel ein Silikat oder ein Tetra(alkyl)silikat, und eines Struktur-steuerndes Mittels in Gegenwart eines wässrigen Lösungsmittels, wodurch eine Hydrolyse des Tetra(alkyl)silikats erfolgt, was zur Bildung eines Sols führt;
(ii) Isolieren eines Feststoffs aus dem Sol und
(iii) Calcinieren des isolierten Feststoffs,
und wobei das Struktur-steuernde Mittel aus einem Surfactant der Formel CₙH₂ₙ₊₁N-(CH₃)₃X, worin X Brom oder Chlor ist und n 8, 10, 12, 14 oder 16 ist, einem Surfactant der Formel CₘH₂ₘ₊₁NH₂, worin m 8, 10, 12, 14, 16 oder 18 ist, einem nicht-ionischen Blockcopolymer-Surfactant der Formel E0₁₀₀PO₃₉EO₁₀₀, worin EO P_{O}-ly(ethylen)oxid darstellt und PO Poly(propylen)oxid darstellt, und einem nicht-ionischen Sterncopolymer-Surfactant der Formel (EO₁₁₃PO₂₂)₂N(CH₂)₂N(PO₂₂EO₁₁₃)₂, worin EO Poly(ethylen)oxid darstellt und PO Poly(propylen)oxid darstellt und Gemischen davon ausgewählt wird.

5. Verfahren gemäß Anspruch 4, wobei das Tetra(alkyl)silikat aus Tetramethylorthosilikat und Tetraethylorthosilikat ausgewählt wird.

6. Verfahren gemäß Anspruch 5, wobei der Siliciumdioxid-Vorläufer ein organischer Vorläufer ist, der ein Tetra(alkyl)silikat in der Form von Tetraethylorthosilikat ist.

7. Verfahren gemäß einem oder mehreren der Ansprüche 4 bis 6, wobei das Calciumsalz aus Calciumnitrat, Calciumfluorid und Calciumchlorid ausgewählt wird.

8. Verfahren gemäß Anspruch 5, wobei das Struktur-steuernde Mittel aus Cetyltrimethylammoniumbromid, einem nicht-ionischen Blockcopolymer-Surfactant der Formel EO₁₀₀PO₃₉EO₁₀₀, worin EO Poly(ethylen)oxid darstellt und PO Poly(propylen)oxid darstellt, einem nicht-ionischen Sterncopolymer-Surfactant der Formel (EO₁₁₃PO₂₂)₂N-(CH₃)₂N(PO₂₂EO₁₁₃)₂, worin EO Poly(ethylen)oxid darstellt und PO Poly(propylen)oxid darstellt und Dodecylamin ausgewählt wird.

9. Verfahren gemäß einem oder mehreren der Ansprüche 4 bis 8, wobei das wässrige Lösungsmittel Wasser und einen C₁-C₄-Alkohol umfasst.

10. Verfahren gemäß einem oder mehreren der Ansprüche 4 bis 9, wobei in Schritt (ii) der Feststoff durch Filtration isoliert wird.

11. Verfahren gemäß einem oder mehreren der Ansprüche 4 bis 9, wobei in Schritt (ii) der Feststoff durch Einengung isoliert wird.

12. Verfahren gemäß einem oder mehreren der Ansprüche 4 bis 11, wobei die Calcinierung in Schritt (iii) bei einer Temperatur im Bereich von 500 bis 800 °C durchgeführt wird.

13. Verfahren zur Bildung von Hydroxylapatit, wobei das Verfahren den Schritt des In-Kontakt-Bringens des Calciumoxid-Siliciumdioxid-Verbundbiomaterials gemäß einem oder mehreren der Ansprüche 1 bis 3 mit Phosphationen bei einem pH im Bereich von 5 bis 10 umfasst.

14. Calciumoxid-Siliciumdioxid-Verbundbiomaterial gemäß einem oder mehreren der Ansprüche 1 bis 3 zur Verwendung bei der Geweberegeneration.

15. Calciumoxid-Siliciumdioxid-Verbundbiomaterial gemäß einem oder mehreren der Ansprüche 1 bis 3 zur Verwendung bei der Zahn- und/oder Knochenregeneration.

16. Calciumoxid-Siliciumdioxid-Verbundbiomaterial gemäß einem oder mehreren der Ansprüche 1 bis 3 zur Verwendung beim Bleichen eines Zahns.

17. Calciumoxid-Siliciumdioxid-Verbundbiomaterial gemäß einem oder mehreren der Ansprüche 1 bis 3 zur Verwendung bei der Behandlung und/oder Prävention von Zahnhyperempfindlichkeit.

18. Geweberegenerations-Zusammensetzung, die ein Calciumoxid-Siliciumdioxid-Verbundbiomaterial gemäß einem oder mehreren der Ansprüche 1 bis 3 umfasst.

19. Knochenregenerations-Zusammensetzung, die ein Calciumoxid-Siliciumdioxid-Verbundbiomaterial gemäß einem oder mehreren der Ansprüche 1 bis 3 umfasst.

20. Zahnregenerations-Zusammensetzung, die ein Calciumoxid-Siliciumdioxid-Verbundbiomaterial gemäß einem oder mehreren der Ansprüche 1 bis 3 umfasst.

21. Eine Zahn-bleichende Zusammensetzung, die ein Calciumoxid-Siliciumdioxid-Verbundbiomaterial gemäß einem oder mehreren der Ansprüche 1 bis 3 umfasst.

22. Zusammensetzung zur Behandlung und/oder Prävention von Zahnhyperempfindlichkeit, wobei die Zusammensetzung ein Calciumoxid-Siliciumdioxid-Verbundbiomaterial gemäß einem oder mehreren der Ansprüche 1 bis 3 umfasst.

## Revendications

1. Biomatériau composite d'oxyde de calcium-silice à l'état amorphe ou à l'état cristallin ayant un diamètre de pores moyen, tel que déterminé par la méthode BET, dans la plage allant de 0,8 à 4 nm, dans lequel la teneur en oxyde de calcium-silice du biomatériau est d'au moins 80 % en poids, et dans lequel le rapport molaire de l'oxyde de calcium sur la silice est d'au moins 0,1, et dans lequel le biomatériau comprend moins de 0,5 % en poids d'ions de phosphate et dans lequel les pores du biomatériau ont une disposition ordonnée.

2. Biomatériau composite d'oxyde de calcium-silice selon la revendication 1, dans lequel le diamètre de pores moyen est dans la plage allant de 1 à 2,7 nm.

3. Biomatériau composite d'oxyde de calcium-silice selon l'une quelconque des revendications précédentes, qui se présente sous la forme d'une poudre.

4. Méthode de préparation d'un biomatériau composite d'oxyde de calcium-silice selon la revendication 1, la méthode comprenant les étapes consistant à :
(i) combiner, en solution, un sel de calcium, un précurseur de silice organique ou inorganique tel qu'un silicate ou un tétra-(alkyl)silicate et un agent directeur de structure en la présence d'un solvant aqueux, moyennant quoi il se produit l'hydrolyse du tétra(alkyl)silicate, aboutissant à la formation d'un sol ;
(ii) isoler un solide du sol ; et
(iii) calciner le solide isolé,
et dans laquelle l'agent directeur de structure est choisi parmi un tensioactif de la formule CₙH₂ₙ₊₁N(CH₃)₃X, dans laquelle X est un brome ou un chlore et n vaut 8, 10, 12, 14 ou 16, un tensioactif de la formule CₘH₂ₘ₊₁NH₂, dans laquelle m vaut 8, 10, 12, 14, 16 ou 18, un tensioactif non ionique bloc-copolymère de la formule EO₁₀₀PO₃₉EO₁₀₀, dans laquelle EO représente l'oxyde de polyéthylène et PO représente l'oxyde de polypropylène, et un tensioactif de copolymère en étoile non ionique de la formule (EO₁₁₃PO₂₂)₂N(CH₂)₂N(PO₂₂EO₁₁₃)₂, dans laquelle EO représente l'oxyde de polyéthylène et PO représente l'oxyde de polypropylène, et des mélanges de ceux-ci.

5. Méthode selon la revendication 4, dans laquelle le tétra(alkyl)silicate est choisi parmi l'orthosilicate tétraméthylique et l'orthosilicate tétraéthylique.

6. Méthode selon la revendication 5, dans laquelle le précurseur de silice est un précurseur organique qui est un tétra(alkyl)-silicate sous la forme d'un orthosilicate tétraéthylique.

7. Méthode selon l'une quelconque ou plusieurs des revendications 4 à 6, dans laquelle le sel de calcium est choisi parmi le nitrate de calcium, le fluorure de calcium et le chlorure de calcium.

8. Méthode selon la revendication 5, dans laquelle l'agent directeur de structure est choisi parmi le bromure de cétyltriméthyl-ammonnium, un tensioactif de non ionique bloc-copolymère de la formule EO₁₀₀PO₃₉EO₁₀₀, dans laquelle EO représente l'oxyde de polyéthylène et PO représente l'oxyde de polypropylène, un tensioactif de copolymère en étoile non ionique de la formule (EO₁₁₃PO₂₂)₂N(CH₂)₂N(PO₂₂EO₁₁₃)₂, dans laquelle EO représente l'oxyde de polyéthylène et PO représente l'oxyde de polypropylène et de la dodécylamine.

9. Méthode selon l'une quelconque ou plusieurs des revendications 4 à 8, dans laquelle le solvant aqueux comprend de l'eau et un alcool en **C₁-C₄.**

10. Méthode selon l'une quelconque ou plusieurs des revendications 4 à 9, dans laquelle dans l'étape (ii) le solide est isolé par filtration.

11. Méthode selon l'une quelconque ou plusieurs des revendications 4 à 9, dans laquelle dans l'étape (ii) le solide est isolé par évaporation.

12. Méthode selon l'une quelconque ou plusieurs des revendications 4 à 11, dans laquelle la calcination dans l'étape (iii) est opérée à une température dans la plage allant de 500 à 800 °C.

13. Méthode de formation d'hydroxylapatite, la méthode comprenant l'étape consistant à mettre en contact le biomatériau composite d'oxyde de calcium-silice selon l'une quelconque ou plusieurs des revendications 1 à 3 avec des ions de phosphate à un pH dans la plage allant de 5 à 10.

14. Biomatériau composite d'oxyde de calcium-silice selon l'une quelconque ou plusieurs des revendications 1 à 3, pour son utilisation pour la régénération tissulaire.

15. Biomatériau composite d'oxyde de calcium-silice selon l'une quelconque ou plusieurs des revendications 1 à 3, pour son utilisation dans la régénération dentaire et/ou osseuse.

16. Biomatériau composite d'oxyde de calcium-silice selon l'une quelconque ou plusieurs des revendications 1 à 3, pour son utilisation dans le blanchiment d'une dent.

17. Biomatériau composite d'oxyde de calcium-silice selon l'une quelconque ou plusieurs des revendications 1 à 3, pour son utilisation dans le traitement et/ou la prévention de l'hypersensibilité dentaire.

18. Composition de régénération tissulaire comprenant un biomatériau composite d'oxyde de calcium-silice selon l'une quelconque ou plusieurs des revendications 1 à 3.

19. Composition de régénération osseuse comprenant un biomatériau composite d'oxyde de calcium-silice selon l'une quelconque ou plusieurs des revendications 1 à 3.

20. Composition de régénération dentaire comprenant un biomatériau composite d'oxyde de calcium-silice selon l'une quelconque ou plusieurs des revendications 1 à 3.

21. Composition de blanchiment dentaire comprenant un biomatériau composite d'oxyde de calcium-silice selon l'une quelconque ou plusieurs des revendications 1 à 3.

22. Composition pour le traitement et/ou la prévention de l'hypersensibilité dentaire, laquelle composition comprend un biomatériau composite d'oxyde de calcium-silice selon l'une quelconque ou plusieurs des revendications 1 à 3.
